# EUROPÄISCHE PATENTSCHRIFT

(11) **EP 1 883 093 B1**
(45) Veröffentlichungstag und Bekanntmachung des Hinweises auf die Patenterteilung: **16.11.2011**
(21) Anmeldenummer: 07111789.9
(22) Anmeldetag: 05.07.2007
(51) Int. Cl.: H01J 35/30, A61B 6/03

(54) **Computertomograph**
CT scanner
Tomodensitomètre

(30) Priorität: 28.07.2006 DE 102006035742; 20.10.2006 DE 102006049630; 20.04.2007 DE 102007019176
(43) Veröffentlichungstag der Anmeldung: 30.01.2008
(73) Patentinhaber: Forster, Jan, 85051 Ingolstadt (DE); Forster, Renate, 85051 Ingolstadt (DE)
(72) Erfinder: Forster, Jan, 85051 Ingolstadt (DE); Müller, Reinhold, 91080 Marloffstein (DE)
(74) Vertreter: Bergmeier, Werner

(56) Entgegenhaltungen:
- DE-A1- 19 515 415
- GB-A- 2 044 985
- US-A- 4 531 226
- US-A- 5 490 193
- US-A1- 2004 234 023
- US-A1- 2005 058 254

## Beschreibung

Die vorliegende Erfindung betrifft einen Computertomographen umfassend zumindest eine stationäre Elektronenquelle zur Erzeugung eines Elektronenstrahles und eine ein Messfeld ringförmig umschließende stationäre Röntgenstrahlenquelle mit einem im wesentlichen ringförmigen Target, mit Mitteln zur Führung des Elektronenstrahles in der Röntgenstrahlenquelle auf einer Kreisbahn koaxial zu dem ringförmigen Target, sowie mit Mitteln zur Ablenkung des Elektronenstrahles in Richtung auf das Target. Hierdurch wird ein sich konzentrisch drehendes Röntgenstrahlenbündel zur Durchstrahlung des Messfeldes aus verschiedenen Richtungen erzeugt. Der Computertomograph umfasst weiterhin einen ringförmig ausgebildeten, stationären Detektor, der aus einer Vielzahl von Detektorelementen besteht, aus deren Messwerten durch einen Rechner ein Bild des Untersuchungsobjektes im Messfeld berechnet wird.

Computertomographiegeräte sind im Stand der Technik in verschiedensten Ausführungen bekannt. Überwiegend eingesetzte Geräte umfassen eine rotierende Röntgenröhre sowie einen rotierenden Detektorring. Die Energie- und Datenübertragung aus den rotierenden Teilen erfolgt über Schleifringe. Hierdurch, sowie durch die hohen Fliehkräfte, welche in den um das Messobjekt rotierenden Teilen auftritt, sind Verkürzungen der Scanzeiten nur sehr begrenzt möglich.

Weiterhin sind bereits Geräte bekannt, bei welchen sich keine mechanischen Komponenten mehr bewegen. Ein derartiges Computertomographiegerät ist beispielsweise aus der DE 42 10 339 A1 bekannt. Das Computertomographiegerät wird hierbei durch eine im wesentlichen ringförmige Struktur gebildet, in welcher ein Elektronenstrahl in einem Vakuumgefäß ringförmig umläuft. Weiterhin ist in der ringförmigen Struktur eine ringförmige Anode angeordnet. Der Elektronenstrahl wird mit Hilfe von Elektronenstrahlführungsmitteln auf der Kreisbahn geführt und mit Hilfe von Extraktionsmitteln in Richtung auf die Anode hin ausgelenkt. Die Kreisbahn des Elektronenstrahls und der Anodenring sind koaxial und koplanar, jedoch auf unterschiedlichen Durchmessern angeordnet. Die Anode ist hierbei als durchgehender Anodenring ausgebildet, welcher durch den Fokus des Elektronenstrahls kontinuierlich abgetastet wird.

Die US 4,531,226 zeigt ein Computertomographiegerät mit einem Elektronenstrahl, der auf ein Target abgelenkt wird. Das Messfeld wird bei dieser Ausführung bogenförmig bzw. im Wesentlichen halbkreisförmig umschlossen, Das Target ist hier ebenfalls als durchgehendes Target ausgebildet, welches kontinuierlichabgetastet wird, um eine umlaufende Röntgenstrahlung zu erzeugen. Um die Wartung und den Austausch des Targets zu erleichtern, sind mehrere kreissegmentförmige Targetringe auf einem Träger angeordnet.

Aus der US 5,490.193 ist ein Computertomograph ohne bewegte mechanische Komponenten bekannt, welcher dae Messfeld bogenförmig umschließt, und mit einem auf einer bogenförmigen Bahn umlaufenden Elektronenstrahl. Ein sägezahnförmiges Target und ein Target mit einzelnen, diskreten Fokusflächen sind aus US 5,490,193 bekannt. Durch eine diskontinuierliche Abtastung der diskreten Fokusflächen wird eine verbesserten Bildqualität erreicht.

Aufgabe der vorliegenden Erfindung ist es, einen Computertomographen vorzuschlagen, welcher eine verbesserte Bildqualität ermöglicht.

Die Aufgabe wird gelöst mit den Merkmalen des Anspruches 1.

Ein Computertomograph umfasst zumindest eine stationäre Elektronenquelle zur Erzeugung eines Elektronenstrahles und eine ein Messfeld ringförmig umschließende stationäre Röntgenstrahlenquelle mit einem im wesentlichen ringförmigen Target, mit Mitteln zur Führung des Elektronenstrahles in der Röntgenstrahlenquelle auf einer Kreisbahn koaxial zu dem ringförmigen Target sowie mit Mitteln zur Ablenkung des Elektronenstrahles in Richtung auf das Target. Weiterhin umfasst der Computertomograh einen ringförmig ausgebildeten stationären Detektor, der aus einer Vielzahl von Detektorelementen besteht. Durch das Auftreffen des abgelenkten Elektronenstrahles auf das Target wird ein sich konzentrisch drehendes Röntgenstrahlenbündel zur Durchstrahlung des Messfeldes aus verschiedenen Richtungen erzeugt. Aus den Messwerten der einzelnen Detektorelemente wird durch einen Rechner ein Bild des Untersuchungsobjektes im Messfeld berechnet. Das ringförmige Target weist eine Vielzahl diskreter Fokusflächen auf, welche diskontinuierlich durch einen Fokus des Elektronenstrahles abtastbar sind. Im Gegensatz zu herkömmlichen Computertomographiegeräten, bei welchen der Fokus kontinuierlich über das Target bzw. die Anodenfläche streicht, und diesem Fokus ein mittlerer Aufenthaltsort auf dem Target bzw. der Anodenfläche zugewiesen wird, wobei ein leicht verschmiertes Bild entsteht, arbeitet der Computertomograph nach der vorliegenden Erfindung mit diskreten Fokusflächen und diskontinuierlicher Abtastung durch den Fokus des Elektronenstrahles. Durch diese Diskretisierung ist es möglich, der in den Detektorelementen gemessenen Strahlung jeweils einen exakten Ort auf der Fokusfläche zuzuordnen. Hierdurch entsteht eine Vielzahl stehender Bilder, welche eine verbesserte Bildqualität liefern. Zugleich ist es durch die exakte Zuordenbarkeit möglich, die Strahlenbelastung bei im Vergleich zum Stand der Technik gleichbleibender oder sogar verbesserter Bildqualität zu verringern.

Die erfindungsgemäße Targetstruktur nutzt eine ungerade Zahl von Targetflächen pro 360°- Umlauf, z. B. 361 oder 721. Diese erfindungsgemäße Anordnung erlaubt sowohl einen "schnelleren" Scan über 180° mit ca. 180 bzw 360 Projektionen (geringere Patientendosis, geringere Ortsauflösung, geringerer Rechenaufwand, etwas unschärferes Ergebnis) als auch einen "langsameren" Scan mit halbem Pitch mit etwa der doppelten Zahl an Projektionen, also 361 bzw. 721. Dies bedeutet für den Patienten etwa die doppelte Dosis, höherer Rechenaufwand, aber auch ein schärferes Ergebnis sowie eine annähernd artefaktfreie Rekonstruktion.

Vorteilhaft ist es, wenn das Target eine gestufte Ringstruktur mit Sägezahnelementen aufweist, deren Teilung zumindest einer 2° Teilung des Vollkreises entspricht. Hierdurch ist es in einfacher Weise möglich, eine Vielzahl diskreter Fokusflächen auf einem Targetring anzuordnen. Die Anzahl der Stufen des Targetringes bestimmt hierbei maßgeblich die Auflösung des Bildes. Im Falle einer 1 ° Teilung weist das Target 361 Stufen auf, es sind jedoch ebenso feinere Abstufungen mit beispielsweise 721 Stufen möglich. Erfindungsgemäß ist hierbei die Zahl der Stufen ungerade, wodurch sich die Anzahl unabhängiger Projektionen verdoppeln lässt.

Alternativ besteht das Target aus einer Vielzahl einzelner Targetelemente, welche jeweils auf einem Sägezahnelement eines gestuften, ringförmigen Trägers angeordnet sind. Hierbei ist ebenfalls eine ungerade Anzahl an Targetelementen auf dem Umfang des Vollkreises verteilt, so dass die Lücken zwischen den einzelnen Targetelementen jeweils durch einen Strahl von der Gegenseite des Targetringes getroffen werden. Wie beschrieben werden durch die Strahlen der Gegenseite des Ringes zusätzliche unabhängige Projektionen erhalten. Zudem ist es hierdurch möglich, die Menge eines vergleichsweise teuren Targetmaterials auf ein Minimum zu reduzieren, und den Träger aus einem geeigneten kostengünstigeren Material herzustellen. Der Träger kann hierbei einteilig mit einem Strahlführungsrohr, in welchem der ringförmige Elektronenstrahl umläuft, ausgebildet sein, oder ein separates Bauteil darstellen.

Nach einer bevorzugten Ausführung der Erfindung sind die einzelnen Targetelemente auswechselbar auf dem Träger angeordnet. Hierdurch ist es möglich die teuren Targetelemente nach Abnutzung gegen neue auszutauschen, wobei der ringförmige Träger weiterverwendet werden kann.

Der Träger und/oder das Strahlführungsrohr ist bevorzugt aus einem wärmeleitenden Material, insbesondere Kupfer hergestellt. Hierdurch ist eine verbesserte Wärmeableitung von den Targetelementen möglich. Zudem ist hier durch eine preisgünstige Herstellung des Trägers möglich.

Vorteilhaft ist es, wenn die diskreten Fokusflächen in einem Winkel von 58° zur Ebene der Kreisbahn des Elektronenstrahles angeordnet sind. Hierdurch muss ein Elektronenstrahl, welcher in einer Ebene parallel zu dem ringförmigen Target umläuft, nurmehr um einen geringen Ablenkwinkel ausgelenkt werden, während im Stand der Technik üblicherweise Ablenkwinkel von 90° erforderlich sind, um ein senkrechtes Auftreffen des Elektronenstrahles auf das Target zu gewährleisten.

Vorteilhaft ist es hierbei, wenn der Träger und/oder das Strahlführungsrohr mehrere separat beaufschlagbare Kühlmlttelkanäle aufweisen. Hierdurch ist eine vorteilhafte Wasserkühlung möglich, mittels welcher die in der Ringstruktur auftretenden Temperaturgradienten gering gehalten werden können. Insbesondere bei großen Durchmessern ist hierdurch eine ausreichende Kühlung erreichbar, so dass keine Problem durch Wärmedehnungen der Bauteile zu erwarten sind.

Eine weitere Ausführungsform der Erfindung sieht vor, dass die kinetische Energie der Elektronen des Elektronenstrahls einstellbar ist. Hierdurch ist es möglich, die Energie der Elektronen an die verschiedensten Messzwecke anzupassen. Die kinetische Energie der Elektronen ist hierbei im Bereich zwischen 80 keV und 1.000 keV einstellbar. Hierdurch ist es möglich, den erfindungsgemäßen Computertomographen für eine Vielzahl von Anwendungen im medizinischen und technischen Bereich einzusetzen. Für technische Messzwecke kommen insbesondere Energien zwischen 150 keV und 1.000 keV in Betracht.

Insbesondere für technische Anwendungen bei der Untersuchung stark absorbierender Objekte kann es aber auch vorteilhaft sein, wenn die kinetische Energie der Elektronen über 1 MeV beträgt. Vorteilhaft hierbei ist, dass bei höheren Erzeugungsspannungen eine insgesamt geringere Schwächung der Strahlung auftritt.

Vorzugsweise sind bei derart hohen Energien der Elektronen die Targetelemente als Durchstrahlungstargets ausgebildet, welche die Bremsstrahlung in Flugrichtung der einfallenden Elektronen abgeben. Der umlaufende Elektronenstrahl wird hierbei derart umgelenkt, dass die Elektronen möglichst radial in Richtung auf das Zentrum des Durchstrahlungstargets einfallen. Der Ablenkwinkel des Elektronenstrahls beträgt in diesem Fall 90°.

Nach einer weiteren Ausführung der Erfindung ist in Strahlrichtung nach den Durchstrahlungstargets jeweils ein Ausgleichskörper angeordnet, um die Intensität der Bremsstrahlung über dem Field of View homogen zu halten.

Eine andere Weiterbildung der Erfindung sieht vor, dass die Durchstrahlungstargets einen Winkel von vorzugsweise 45° zur Kreisbahn des Elektronenstrahls aufweisen. Die Ausgleichskörper sind hierbei in Form eines Dreikants mit einem Schnittwinkel von vorzugsweise 45° ausgebildet. Hierdurch kann eine dichte Anordnung der Durchstrahlungstargets erreicht werden, wobei dennoch je Durchstrahlungstarget ein Ausgleichskörper ohne Platzprobleme angeordnet werden kann.

Um nach dem Einspeisen des Elektronenstrahls diesen auf einer Kreisbahn in der Röntgenstrahlenquelle führen zu können, ist es vorteilhaft, wenn die Mittel zur Führung des Elektronenstrahls durch Spulen oder Umlenkmagnete gebildet sind. Vorteilhaft ist es weiterhin, wenn die Anzahl der Mittel zur Führung der Anzahl der Sägezahnelemente entspricht. Hierbei erhält jeder Zahn am Target einen eigenen Umlenkmagneten. Je nach Anzahl der Umlenkmagnete, welche in der Röntgenstrahlenquelle angeordnet sind, müssen diese jeweils den Strahl um eine exakte Anzahl von Winkelgraden biegen. Nach einer bevorzugten Ausführung weist das Target 721 Zähne auf und es sind 721 Umlenkmagnete angeordnet, welche den Strahl jeweils um 0,5° umlenken.

Weiterhin ist es vorteilhaft, wenn die Mittel zur Führung und die Mittel zur Ablenkung des Elektronenstrahls nacheinander einzeln ansteuerbar sind. Der Elektronenstrahl trifft hierbei nacheinander exakt auf einen Punkt auf jeder Fokusfläche. Daneben können hierdurch auch unterschiedliche Haltezeiten des Elektronenstrahls auf verschiedenen Fokusflächen realisiert werden.

Nach einer besonders vorteilhaften Weiterbildung der Erfindung sind die Mittel zur Ablenkung des Elektronenstrahles durch Kickvorrichtungen an den Mitteln zur Führung des Elektronenstrahles gebildet. Diese können jeweils magnetisch oder auch elektrostatisch ausgeführt sein. Hierdurch kann in einfacher Weise sowohl die Führung des Elektronenstrahls wie auch die Ablenkung des Elektronenstrahls in Richtung auf das Target durch jeweils ein gemeinsames Element realisiert werden. Sind die Mittel zur Führung des Elektronenstrahls durch Umlenkmagnete gebildet, lässt sich eine besonders platzsparende Ausführung des Computertomographen erreichen.

Vorteilhaft ist es weiterhin, wenn der Ablenkwinkel der Kickvorrichtung 32° beträgt. Selbstverständlich sind auch andere Ablenkwinkel denkbar, welche jeweils von dem Winkel der Fokusflächen zur Ebene der Kreisbahn des Elektronenstrahls abhängig sind. Vorteilhaft ist hierbei, dass eine Ablenkung des Elektronenstrahls um 90° wie im Stand der Technik nicht erforderlich ist.

Eine vorteilhafte Weiterbildung der Erfindung sieht vor, dass der Ablenkwinkel der Kickvorrichtungen verstellbar ist. So können auch mehrere Targetelemente mit einer Umlenkvorrichtung zusammengefasst werden. Die Anzahl der Kickvorrichtungen kann hierdurch reduziert werden, wobei zugleich mehr Bauraum für die Konstruktion der Umlenkung und Fokussierung entsteht. Vorteilhaft ist es, wenn der Kickvorrichtung eine Elektronenlinse zugeordnet ist. Der Elektronenstrahl wird hierbei nach Umlenkung auf den Fokus so geformt, dass sich eine optimale Fokusgröße ergibt.

Weiterhin ist es vorteilhaft, wenn die Fokusflächen einen Anschnittwinkel von vorzugsweise 12° zur Achse des ringförmigen Targets aufweisen. Auch hierdurch kann der optische Fokus klein gehalten werden. Je nach Ausführung sind jedoch auch andere Anschnittwinkel vorteilhaft.

Nach einer Weiterbildung der Erfindung ist es vorteilhaft, wenn der Fokus des Elektronenstrahls auf den diskreten Fokusflächen verstellbar ist. Die Verstellung des Fokus kann mittels der Kickvorrichtungen sowie elektronenoptischer Elemente realisiert werden. Um die Korrosion des Targets möglichst gering zu halten, kann beispielsweise der Fokus turnusmäßig auf dem Target um jeweils einige Fokusbreiten verstellt werden. Ebenso sind auch Ausführungen des Fokus als Strichfokus und/oder Springfokus möglich, wobei auch zusätzliche Umlenkvorrichtungen für den Elektronenstrahl vorgesehen sein können.

Vorteilhaft ist es, wenn der Fokus des Elektronenstrahls als Strichfokus ausgebildet ist. Die Wärmelast auf dem Target kann hierdurch auf eine größere Fläche verteilt werden, so dass auch bei einer höheren Strahlungsleistung dennoch kurze Scanzeiten realisierbar sind. Vorteilhaft ist es beispielsweise, wenn der Strichfokus eine Dimension von 1 mm * 10 mm aufweist. Bei einem Anschnittwinkel von 6° ergibt sich somit ein virtueller Fokus von ca. 1mm * 1 mm.

Der Strichfokus kann hierbei kontinuierlich auf dem Target bewegt werden, was bei der Rekonstruktion des Bildes zu berücksichtigen ist. So kann der Fokus beispielsweise über eine Länge von 20 mm verwischt werden, um die instantane Wärmelast auf den Targets zu reduzieren.

Alternativ oder zusätzlich kann der Fokus des Elektronenstrahls als Springfokus ausgebildet sein. Der Springfokus kann ebenfalls mittels elektronenoptischer Elemente realisiert werden. Auch durch Ausbildung eines Springfokus kann die instantane lokale Wärmelast auf den Targets reduziert werden. Zudem besteht hierdurch die Möglichkeit, zusätzliche unabhängige Projektionen zu erhalten, wodurch je nach Ausführung ein Effekt zusätzlicher Detektorzeilen erreicht wird, ohne dass hierzu der Detektor tatsächlich mehr Zeilen aufweisen müsste, oder aber die Auflösung erhöht wird.

Vorteilhaft ist es, wenn dem Springfokus genau eine Zeile des Detektors zugeordnet ist, wobei der Fokus an mehrere Plätze der Zeile springt. Der Fokus kann hierbei an zwei oder auch mehr Plätze der Detektorzeile springen.

Nach einer anderen Weiterbildung ist es vorteilhaft, wenn dem Fokus mehrere Zeilen des Detektors zugeordnet sind. Der Fokus kann hierbei, wie oben für eine Detektorzeile beschrieben, an mehrere Plätze mehrerer Zeilen springen, oder auch in mehreren Detektorzeilen über eine bestimmte Länge verwischt werden. Hierdurch kann die Anzahl der unabhängigen Projektionen während eines Umlaufs erhöht werden, ohne dass hierzu mehr Detektorzeilen erforderlich sind. Zugleich können die Zeiten für einen Umlauf und damit die gesamte Scanzeit vorteilhaft verkürzt werden.

Da bei einem Springen bzw. Verschmieren des Fokus in mehrere Zellen die Anzahl der Projektionsrichtungen erhöht werden kann, ist es möglich, die Anzahl der einzelnen Targetelemente zu verringern, wobei dennoch eine ausreichende Anzahl von Projektionen bei guter Auflösung erhalten wird. Die Umlaufzeit kann hierdurch weiterhin verkürzt werden, wobei eine Umlaufdauer von 50 ms und darunter ermöglicht wird.

Eine besonders vorteilhafte Weiterbildung sieht vor, dass die Photonenfluenz einstellbar ist. Dies kann beispielsweise in bekannter Weise durch Veränderung des Stromes des Elektronenstrahls erfolgen. Insbesondere ist es vorteilhaft, wenn die Photonenfluenz für jede Fokusfläche Individuell einstellbar ist. Dies ermöglicht eine optimale Anpassung der Photonenfluenz an die jeweiligen Messbedingungen. Vorzugsweise wird hierbei die Photonenfluenz durch Regelung der Haltezeit des Elektronenstrahls am einzelnen Fokus eingestellt. Der Computertomograph kann hierdurch immer mit maximalem Strom und voller Leistung betrieben werden, da die Fluenz am jeweiligen Fokus lediglich durch die Haltezeit gesteuert ist.

Vorteilhaft ist es, wenn der ringförmige Detektor axial versetzt zu dem ringförmigen Target angeordnet ist. Um den Detektor optimal in Bezug auf den Strahlengang anzuordnen, kann dieser auch leicht geneigt angeordnet werden, um ein annähernd senkrechtes Auftreffen der Röntgenstrahlung sicherzustellen.

Weitere Vorteile der Erfindung werden anhand der nachfolgend ausgeführten Beispiele erläutert. Es zeigen:
- **Figur 1**: eine schematische Schnittdarstellung eines erfindungsgemä- ßen Computertomographen,
- **Figur 2**: eine Schnittdarstellung durch die ringförmige Röntgenstrahl- quelle und den zugeordneten, ringförmigen Detektor,
- **Figur 3**: eine schematische Darstellung eines ringförmigen Targets mit Sägezahnelementen in einer Draufsicht und einer Seitenan- sicht,
- **Figur 4**: eine schematische Schnittdarstellung eines Strahlführungsroh- res mit Kühlmittelkanälen,
- **Figur 5**: eine schematische Darstellung der ringförmigen Struktur eines erfindungsgemäßen Computertomographen für technische An- wendungen,
- **Figur 6**: eine schematische Darstellung der Anordnung von Durchstrah- lungstargets eines Computertomographen für technische An- wendungen und
- **Figur 7**: eine schematische Darstellung der Anordnung von Durchstrah- lungstargets und Ausgleichskörper.

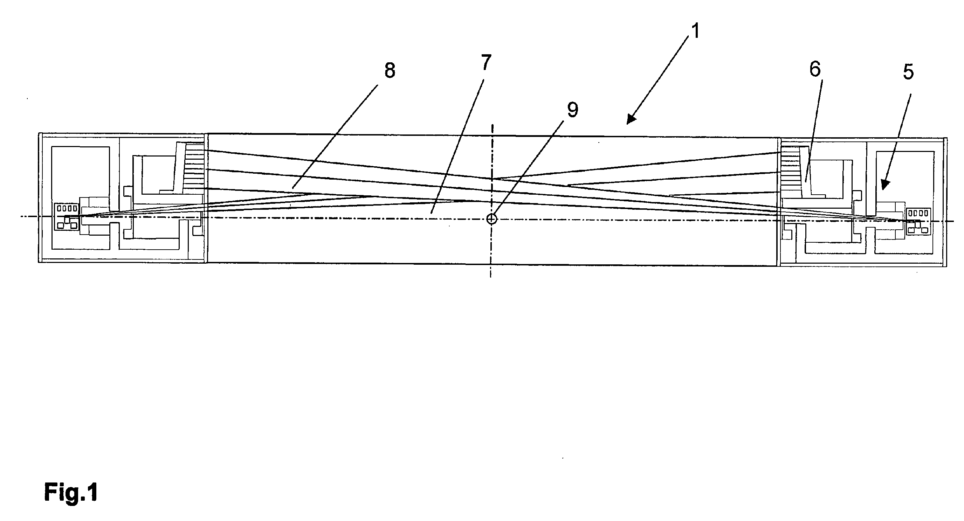

Figur 1 zeigt einen erfindungsgemäßen Computertomographen 1 in einer schematischen Schnittdarstellung durch die Symmetrieachse der Struktur. Der Computertomograph 1 besteht aus einer ringförmigen Struktur, in welcher die wesentlichen Bauteile des Computertomographen wie die Röntgenstrahlenquelle 5 und ein ringförmiger Detektor 6 angeordnet sind. Die ringförmige Struktur umschließt hierbei ein Messfeld 7, welches durch ein sich konzentrisch drehendes Röntgenstrahlenbündel 8 durchstrahlt werden kann. Im Zentrum der Ringstruktur befindet sich das Isozentrum 9 der Anordnung, in welchem ein Messobjekt (hier nicht dargestellt) platziert werden kann. Die Röntgenstrahlen treffen schließlich auf einen gegenüberliegend angeordneten Detektor 6, welcher in bekannter Weise aus einer Vielzahl aneinandergereihter Detektorelemente besteht, auf. Der Detektor 6, die Röntgenstrahlenquelle 5 sowie die hier nicht dargestellte Elektronenquelle zur Erzeugung eines Elektronenstrahles sind hierbei stationär ausgebildet, so dass der Computertomograph 1 keinerlei bewegte Bauteile aufweist.

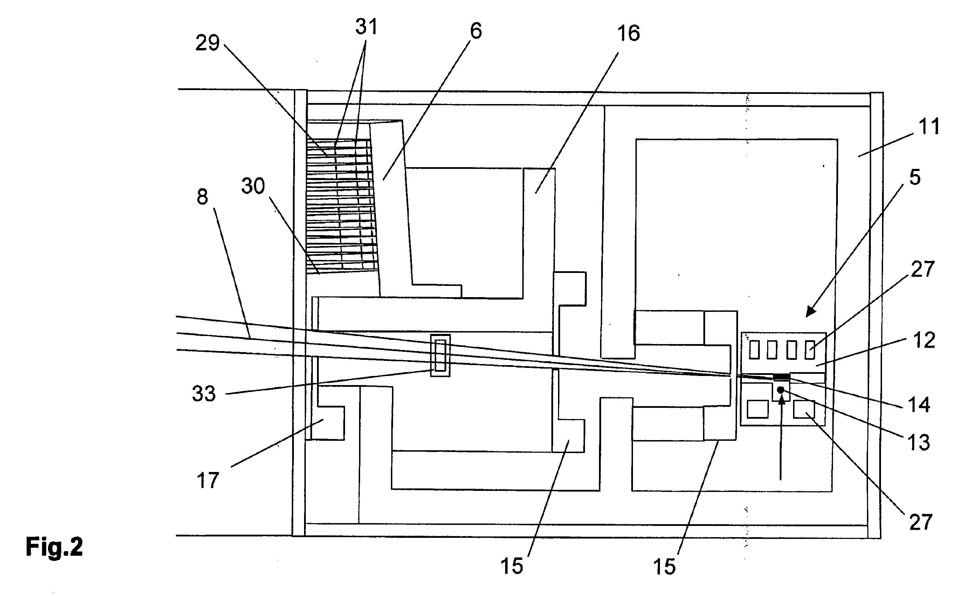

Figur 2 zeigt eine schematische Darstellung des Schnittes im Bereich der Röntgenstrahlenquelle 5 und des Detektors 6 in einer Detaildarstellung. Der Computertomograph 1 weist einen ringförmigen, torsionssteifen Tragrahmen 11 auf, an welchem die Bauteile des Computertomographen angeordnet sind. An dem Tragrahmen 11 ist ein Strahlführungsrohr 12 angeordnet, in welchem ein Elektronenstrahl 13 auf einer Kreisbahn umläuft. Der Elektronenstrahl 13 ist durch Mittel zur Führung des Elektronenstrahles auf einer Kreisbahn gehalten. An dem Strahlführungsrohr 12 ist weiterhin ein im wesentlichen ringförmiges Target 14 angeordnet, in welchem beim Auftreffen des umgelenkten Elektronenstrahles 13 in bekannter Weise die Röntgenstrahlung erzeugt wird. Das Target ist an der Außenwand des Strahlführungsrohres 12 angeordnet. Das erzeugte Röntgenstrahlenbündel 8 wird für eine optimale Strahlführung mehrfach durch Blenden, welche als Festblendenringe 15 oder auch verstellbare Blenden 17 ausgeführt sein können, geformt. Axial versetzt zu dem ringförmigen Strahlführungsrohr 12 mit Target 14 ist ein feststehender Detektor 6, welcher ebenfalls ringförmig ausgebildet ist, angeordnet. Um fehlerhafte Auswertungen durch Streustrahlung zu vermeiden, ist zwischen dem Detektorring 6 und dem Target 14 eine geräteinteme Abschirmung 16 angeordnet.

Der Detektor 6 wird entsprechend der Neigung des Zentralstrahles des Strahlenbündels leicht geneigt angeordnet, um ein annähernd senkrechtes Auftreffen der Röntgenstrahlung sicherzustellen.

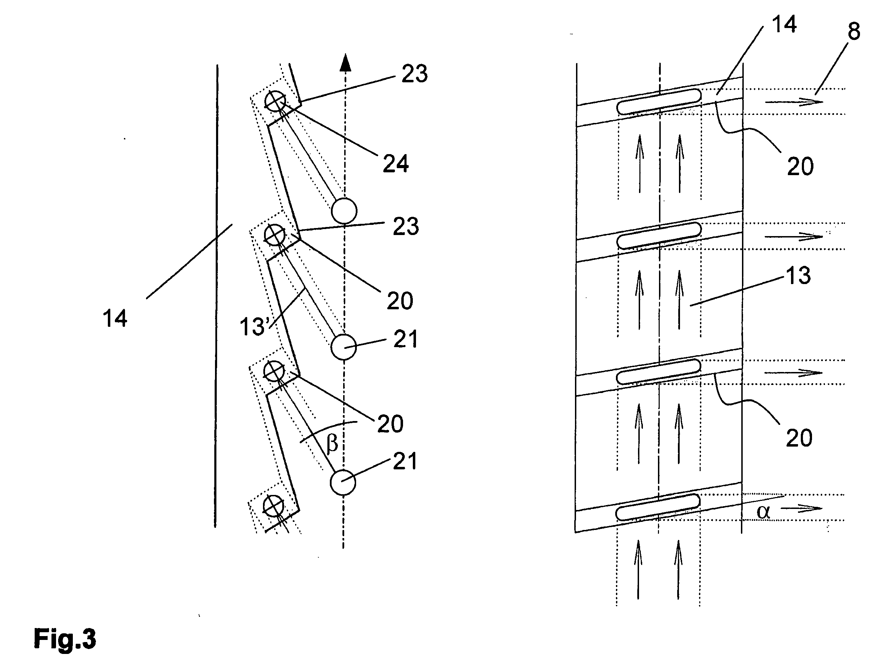

Das im wesentlichen ringförmige Target 14 weist eine Vielzahl diskreter Fokusflächen 20 auf (Figur 3). Parallel und konzentrisch zu dem ringförmigen Target 14 läuft in dem Strahlführungsrohr (hier nicht dargestellt) ein Elektronenstrahl 13 auf einer Krelsbahn um. Der Elektronenstrahl 13 ist durch Mittel zur Führung des Elektronenstrahles auf der Kreisbahn gehalten. Durch Mittel 21 zur Ablenkung des Elektronenstrahles 13 wird der Elektronenstrahl 13 jeweils auf die Fokusflächen 20 gelenkt. Die Abtastung des Targets 14 durch den Elektronenstrahl 13' erfolgt somit nicht kontinuierlich umlaufend, sondern diskontinuierlich an genau festlegbaren Punkten. Hierdurch ist es möglich, die Bildqualität Im Vergleich zum Stand der Technik zu verbessern, da der am Detektor 6 detektierten Strahlung jeweils ein exakter Ort auf der Fokusfläche 20 zugeordnet werden kann. Die Fokusflächen 20 sind bevorzugt in einem Winkel zur Ebene der Kreisbahn angeordnet, in der hier gezeigten Darstellung 58°. Hierdurch muss der ringförmig umlaufende Elektronenstrahl 13 jeweils lediglich um den Ablenkwinkel β, vorliegend 32°, abgelenkt werden. Das erzeugte Röntgenstrahlenbündel 8 wird hierbei im wesentlichen senkrecht zu dem eintretenden Elektronenstrahl 13' abgestrahlt, wie in der Seitenansicht (rechte Darstellung) der Figur 3 erkennbar. Durch den Winkel β der Fokusflächen kann der Querschnitt des abgestrahlten Röntgenstrahlenbündels 8 vorteilhaft reduziert werden.

Wie in der Seitenansicht der Figur 3 erkennbar, weisen die Fokusflächen 20 zudem einen Anschnittwinkel α zur Achse des ringförmigen Targets auf. In der hier gezeigten Darstellung beträgt der Anschnittwinkel α 12°. Hierdurch verkürzt sich der Querschnitt des abgestrahlten Bündels auf ca. 1/5, wie der Zeichnung zu entnehmen ist.

Wie in der Draufsicht (linke Darstellung) der Figur 3 ersichtlich, weist das Target 14 eine gestufte Ringstruktur mit Sägezahnelementen 23 auf. Die Teilung der Sägezahnelemente 23 entspricht hierbei zumindest einer 2º-Tellung des Vollkreises. Anstelle eines kontinuierlich laufenden Bildes wird hierbei eine Vielzahl stehender aneinandergereihter Bilder erhalten, was eine Verbesserung der Qualität der Rekonstruktion ermöglicht. Zugleich ist es hierdurch möglich, die Strahlenbelastung für den Patienten zu reduzieren.

Das Target 14 besteht vorzugsweise aus einer Wolframlegierung; es ist jedoch ebenso möglich, lediglich ausreichend große Wolframplättchen, welche eine Vielzahl einzelner Targetelemente bilden, auf jeweils einem Sägezahnelement 23 eines gestuften ringförmigen Trägers anzuordnen. Der Träger kann an dem Strahlführungsrohr 12 angebracht sein oder aber einteilig mit diesem ausgebildet sein. Die einzelnen Targetelemente können auswechselbar auf dem Träger angeordnet sein, so dass diese nach Abnutzung ausgewechselt werden können, während der Träger weiterverwendet werden kann. An dem Träger können auch Halterungen für die Targetelemente vorgesehen sein, welche gleichzeitig eine Lochblende sowie seitliche Abschirmungen aufweisen. Der Austausch der Targetelemente kann hierdurch sehr einfach gestaltet werden. Hierdurch sind deutliche Kosteneinsparungen möglich, da der Verbrauch an kostspieligem Wolfram geringgehalten wird.

Bevorzugt weist das Target 14 361 oder 721 Stufen auf, wobei bei mehr Stufen eine höhere Auflösung der gewonnen Bilder zu erwarten ist. Wie in Figur 3 ersichtlich, ist je Sägezahnelement 23 jeweils ein Mittel 21 zur Ablenkung des Elektronenstrahles vorgesehen. Der Träger des Targets 14 kann bevorzugt aus einem kostengünstigen Kupfermaterial ausgeführt werden, welches zudem für eine gute Wärmeableitung aus dem Bereich des Targets 14 sorgt.

Um die Korrosion des Targets 14 gering zu halten, ist es vorteilhaft, den Fokus 24 auf der Fokusfläche 20 um einige Fokusbreiten wandern zu lassen. Dies kann durch gezielte Ansteuerung der Mittel zur Ablenkung 21 erreicht werden. Vorzugsweise ist hierzu den Mitteln zur Ablenkung 21 eine Elektronenlinse zugeordnet. Hierdurch kann der Abbrand des Targetmaterials reduziert und die Lebensdauer verlängert werden.

Wie in Figur 2 ersichtlich, weist das Strahlführungsrohr 12 Kühlmittelkanäle 27 auf, welche durch ein Kühlmedium, insbesondere Wasser, durchströmbar sind. Hierdurch lässt sich eine besonders gute Wärmeabfuhr aus dem Bereich des Targets 14 realisieren. Durch die umlaufende Wasserkühlung kann zugleich der Temperaturgradient auf dem Radius gering gehalten werden. Insbesondere bei größeren Durchmessern kann es auch vorteilhaft sein, wenn die Kühlkanäle 27 separat beaufschlagbar sind.

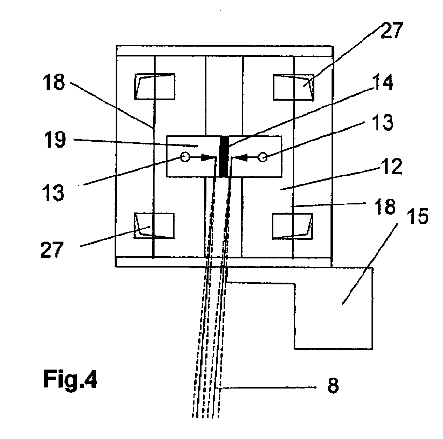

Fig. 4 zeigt ebenfalls ein Strahlführungsrohr 12 mit Kühlmittelkanälen 27. Das Strahlführungsrohr ist hierbei aus mehreren scheibenförmigen Ringelementen zusammengesetzt, in welche die Kühlmittelkanäle 27 als Nuten eingebracht sind. Die Trennlinie 18 ist hierbei in den Bereich der neutralen Faser gelegt, um Probleme durch Verzug zu vermeiden. Zwischen den paarweise zusammengesetzten Ringelementen entsteht ein Hohlraum 19, in welchem im Vakuum der Elektronenstrahl 13 umläuft und das Target 14 angeordnet ist.

Die hier nicht dargestellte Elektronenquelle ist vorzugsweise als Elektronenkanone ausgeführt und speist den Elektronenstrahl 13 mittels elektronenoptischer Elemente tangential ein. Die Elektronenkanone ist hierbei außerhalb der Röntgenstrahlenquelle angeordnet und kann daher beliebig ausgeführt werden. Auch das Anbringen einer zweiten Elektronenkanone ist daher ohne Probleme möglich. Die Elektronenkanone kann hierbei nach Art einer thermischen Kathode oder auch als Elektronenbeschleuniger ausgeführt sein. Für medizinische Anwendungen weisen die Elektronen kinetische Energien von etwa 80 bis 150 keV auf. Ebenso ist jedoch auch die Untersuchung großer und kleiner technischer Bauteile mit kinetischen Energien bis zu 1.000 keV möglich. Für bestimmte technische Anwendungen können jedoch auch Energien von über 1 MeV vorgesehen sein. Als Elektronenquelle kann dann ein Linearbeschleuniger oder ein Microtron-Beschleuniger vorgesehen sein.

Wie in Figur 2 ersichtlich, ist dem Detektor 6 ein Streustrahlenraster 29 mit Ringlamellen 30 zugeordnet. Hierdurch kann ein Rauschen vermindert werden und die Bildqualität erheblich verbessert werden. Die Ringlamellen 30 können hierbei in einfacher Weise parallel zueinander angeordnet sein. In einer bevorzugten Ausführung sind die einzelnen Ringlamellen 30 jeweils in einem bestimmten Winkel exakt auf den Fokus 24 am gegenüberliegenden Target 14 ausgerichtet. Besonders vorteilhaft ist es hierbei, wenn das Streustrahlenraster 29 zwischen den Ringlamellen 30 in Umlaufrichtung des Elektronenstrahles 13 elektromechanisch verstellbare Querlamellen 31 aufweist. Das Streustrahlenraster 29 ist hierbei nach Art eines Rechteckgitters ausgeführt. Die Querlamellen 31 können hierdurch ebenfalls genau auf den Strahlenfokus ausgerichtet werden. Die elektromechanische Steuerung kann beispielsweise in Piezotechnologie ausgeführt werden.

Durch die verstellbaren Querlamellen 31 ist es möglich, die Röntgenstrahlen 8 in optimaler Weise auf den Detektor 6 zu leiten. Der Verstellwinkel der Querlamellen ist hierbei vom Durchmesser des Messfeldes abhängig. In Weiterentwicklung der Erfindung ist es denkbar, die verstellbaren Querlamellen 31 um +/- 90° zu verstellen, um sie ganz zuzuklappen. Hierdurch können einzelne Detektorelemente des Detektors 6 vor zu großer Photonenfluenz geschützt werden; insbesondere werden Detektoren außerhalb des Messkegels stumm gestellt.

Mit dem Computertomographen 1 mit einer Vielzahl diskreter Fokusflächen 20 ist es weiterhin möglich, die Photonenfluenz für jede Fokusfläche 20 individuell einzustellen. Hierdurch ist es möglich, die Photonenfluenz winkelabhängig und abhängig von der Absorption zu modulieren. In herkömmlichen Computertomographiegeräten erfolgt die Modulation der Photonenfluenz durch Modulation des Röhrenstromes. Durch die erfindungsgemäße Diskretisierung des Targets 14 sowie die diskontinuierliche Abtastung ist es mit dem erfindungsgemäßen Computertomographen 1 möglich, die Photonenfluenz allein durch Regelung der Haltezeit des Elektronenstrahls 13 am einzelnen Fokus 24 einzustellen. Der Computertomograph 1 kann hierbei immer mit maximalem Strom bei voller Leistung betrieben werden.

Die Haltezeit des Elektronenstrahls wird bevorzugt durch Messung der Photonenfluenz am jeweiligen Fokus 24 geregelt. Weiterhin ist es möglich, die Haltezeit in Abhängigkeit eines Dosiseintrages der dem jeweiligen Fokus 24 zugeordneten Detektorelemente zu regeln. Der Elektronenstrahl 13 wandert in diesem Fall erst weiter, wenn ein ausreichender Dosiseintrag erfolgt ist.

Zur Messung der Photonenfluenz ist, wie in Figur 2 gezeigt, eine Durchstrahlungskammer 33 außerhalb der Röntgenstrahlenquelle 5 angeordnet. An Stelle einer Vielzahl von Einzelkammem 33 je Fokusfläche 20 ist es jedoch auch möglich, nur eine große ringförmige Kammer einzusetzen. Da zeitgleich immer nur jeweils ein Fokus 24 Röntgenstrahlung 8 abstrahlt, ist bei entsprechend gewählter Zeitkonstante für die Messung das Signal immer exakt einer Fokusfläche 20 zuordenbar. Die Modulation der Photonenfluenz in Abhängigkeit des Dosiseintrages am Detektor 6 sowie der jeweiligen Ladung am individuellen Fokus 24 ermöglicht es, die Strahlendosis optimal an das Messobjekt anzupassen und hierdurch die Strahlenbelastung für Patienten erheblich zu reduzieren.

Der Computertomograph 1 ist somit sowohl an das Messobjekt als auch verschiedenste Messaufgaben im technischen und medizinischen Bereich anpassbar. Da in dem Computertomograph 1 keinerlei bewegte Teile vorhanden sind, ist der Computertomograph 1 besonders vielfältig einsetzbar. Da an Stelle einer herkömmlichen Röntgenröhre die Elektronen in einer außerhalb der Röntgenstrahlenquelle 5 angeordneten Elektronenquelle erzeugt werden, können Elektronenstrahlen 13 verschiedenster kinetischer Energien erzeugt werden. Für technische Messzwecke können somit Elektronen mit kinetischen Energien zwischen 150 keV und 1.000 keV zur Verfügung gestellt werden.

Die Ortsauflösung des rekonstruierten "Bildes" der Schwächungswerte des Objektes hängt von einer ganzen Reihe von Faktoren ab. Einer davon ist die Abtastrate pro Umlauf. Es genügt dabei eine Abtastung um 180°, da eine Abtastung in Gegenrichtung im Prinzip dieselbe physikalische Information enthält. Im Falle der diskretisierten Abtastung in z. B. ungefähr 1°-Schritten ergeben sich somit 180 Projektionen des Objektes, die zur "Bildrekonstruktion" herangezogen werden können. Durch die erfindungsgemäße Anordnung des Targetrings, kann aber die Zahl der unabhängigen Projektion verdoppelt werden.

Die erfindungsgemäße Targetstruktur nutzt eine ungerade Zahl von Targetflächen pro 360°- Umlauf, z. B. 361 oder 721. Diese erfindungsgemäße Anordnung erlaubt sowohl einen "schnelleren" Scan über 180° mit ca. 180 bzw 360 Projektionen (geringere Patientendosis, geringere Ortsauflösung, geringerer Rechenaufwand, etwas unschärferes Ergebnis) als auch einen "langsameren" Scan mit halbem Pitch mit etwa der doppelten Zahl an ProJektionen, also 361 bzw. 721. Dies bedeutet für den Patienten etwa die doppelte Dosis, höherer Rechenaufwand, aber auch ein schärferes Ergebnis sowie eine annähernd artefaktfreie Rekonstruktion.

Es liegt ebenfalls im Rahmen der Erfindung zusätzlich die gesamte Ringstruktur- 2 in kleinen Schritten im Bereich des Winkelabstandes der Targets, z. B. 1º zu drehen, um die Abtastung pro Umlauf im Prinzip beliebig zu steigem. Hierdurch kann beispielsweise bei einem Target mit 361 Stufen in einem Umlauf dennoch eine sehr viel höhere Auflösung erreicht werden. Diese Ausführung kann bei sehr hohen Anforderungen in der Materialuntersuchung von Vorteil sein.

Dadurch, dass eine ungerade Anzahl an Targetelementen auf dem Umfang des Vollkreises verteilt ist, werden die Lücken I zwischen den einzelnen Targetelementen jeweils durch einen Strahl von der Gegenseite des Targetringes getroffen. Wie beschrieben werden durch die Strahlen der Gegenseite des Ringes zusätzliche unabhängige Projektionen erhalten.

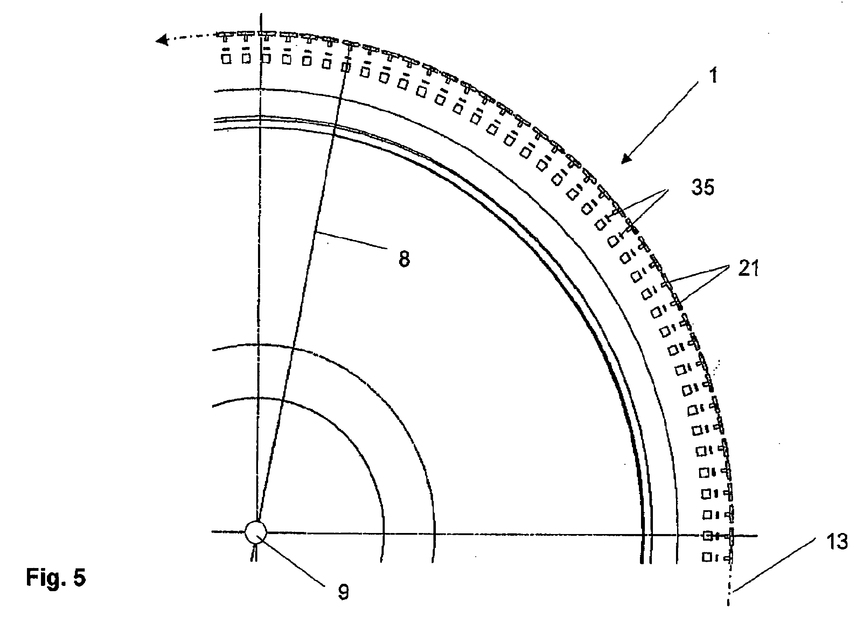

Figur 5 zeigt eine schematische Darstellung eines erfindungsgemäßen Computertomographen 1 für technische Anwendungen in einer Ansicht auf die ringförmige Struktur des Computertomographen 1. Die Darstellung zeigt die Ringstruktur des Computertomographen 1, wobei die einzelnen Targetelemente als Durchstrahlungstargets 35 ausgebildet sind. Für die Materialprüfung größerer, schwererer oder stark absorbierender Objekte sind im Gegensatz zu den medizinischen Anwendungen kinetische Energien im Bereich von 1 bis 3 Megaelektronenvolt erwünscht. Diese können mittels eines entsprechenden Beschleunigers, welcher als Linearbeschleuniger oder Microtronbeschleuniger ausgeführt ist, in einer externen Elektronenkanone erzeugt werden und beispielsweise, wie beschrieben, tangential in das Strahlführungsrohr eingespeist werden.

Da bei derart hohen Energien das Maximum der Bremsstrahlung in Flugrichtung der auftreffenden Elektronen auftritt, sind hierbei die Durchstrahlungstargets 35 derart angeordnet, dass die erzeugte Röntgenstrahlung 8 in Richtung auf das Isozentrum 9 des Computertomographen 1 abgegeben wird. Der Elektronenstrahl 13 läuft hierbei nicht parallel zum Targetring, sondern auf einer Kreisbahn mit einem größeren Radius als der Targetring um. Die Ablenkvorrichtungen 21 sind hierbei derart ausgebildet, dass der Elektronenstrahl 13 vor dem Target jeweils um 90° umgelenkt wird, damit die Elektronen möglichst radial in Richtung auf das Zentrum einfallen. Im Gegensatz zu den vorbeschriebenen Ausführungen wird hierbei nur ein Punktfokus erzeugt.

In Strahlrichtung nach den Durchstrahltargets 35 sind jeweils Ausgleichskörper 36 angebracht, welche beispielsweise aus Edelstahl bestehen. Diese halten die Intensität der Röntgenstrahlung über dem Field of View annähernd homogen. Um die Ausgleichskörper 36 klein zu halten und ohne Überlappung unterzubringen, werden sie auf einem Ring (hier nicht dargestellt) dicht hinter den Durchstrahltargets 35 angebracht, wobei der Ring eine Art Sägezahnstruktur aufweist.

Nach einer weiteren, hier nicht dargestellten, Ausführung der Erfindung kann es auch vorteilhaft sein, mehrere Durchstrahltargets 35 (beispielsweise drei Targets) mit einer Umlenkvorrichtung 21 zusammenfassen. Der Elektronenstrahl 13' wird hierbei abwechselnd aus der reinen 90° Umlenkung auch auf die anderen Targets hin gelenkt. Hierdurch kann die Anzahl der Ablenkvorrichtungen 21 bzw. Kickvorrichtungen reduziert werden, so dass für die Konstruktion der Umlenkung und Fokussierung mehr Platz zur Verfügung steht.

Für den Betrieb mit derart hohen Energien müssen die Detektoren 6 entsprechend angepasst werden, wobei vorzugsweise Festkörperdetektoren angewendet werden. Die weiteren Bauelemente sind hier nicht dargestellt und entsprechen im Wesentlichen den bereits beschriebenen für medizinische Anwendungen mit geringeren Energien. Jedoch ist eine verbesserte Abschirmung gegen Streustrahlung erforderlich, wozu ein Streustrahlungsraster 29, wie in Figur 2 beschrieben, angeordnet werden kann, welches vorzugsweise aus starren Metallringen, beispielsweise aus Wolfram besteht. Ebenso ist auch die Abschirmung der gesamten Struktur gegen die Durchlassstrahlung entsprechend zu gestalten. Weiterhin sind auch die Blenden an die höhere Durchdringung der harten Strahlung anzupassen, so dass vorzugsweise Wolframblenden eingesetzt werden.

Der Computertomograph 1 kann hierbei in seinen Dimensionen auch an die Untersuchung sehr großer Objekte angepasst werden. So kann der Durchmesser des ringförmigen Targets 14 auch in Größenordnungen von bis zu 8 m liegen, wobei beispielsweise 601 einzelne Targetelemente auf dem Umfang angeordnet sind. Hierbei besteht auch die Möglichkeit, das ringförmige Target 14 in Form eines Polygonzugs aus einzelnen, geraden Teilsegmenten auszubilden, da diese insbesondere bei den genannten Dimensionen leichter herzustellen sind. Hierbei ist dann jeweils eine bestimmte Anzahl einzelner Targetelemente auf einem geraden Teilsegment angeordnet. Die einzelnen Teilsegmente werden schließlich vakuumdicht miteinander verbunden.

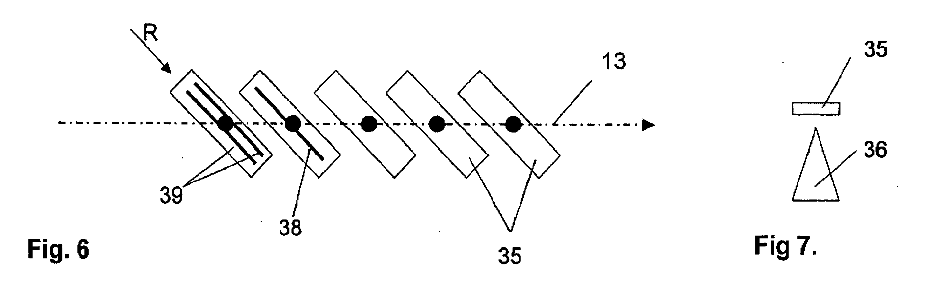

Figur 6 zeigt eine schematische Darstellung der Anordnung von Durchstrahlungstargets 35 eines Computertomographen 1 für technische Anwendungen. Wie unter Figur 5 beschrieben, sind für technische Anwendungen mit hohen Elektronenenergien die einzelnen Targetelemente als Durchstrahlungstargets 35 ausgebildet. Der Elektronenstrahl 13 läuft hierbei wie beschrieben auf einer Kreisbahn mit einem größeren Radius als dem der Durchstrahlungstargets 35 um und wird von dort jeweils um 90° in Richtung auf das Isozentrum 9 der Vorrichtung umgelenkt. In der vorliegenden Darstellung ist dies durch die Punkte auf den Durchstrahlungstarget 35 angedeutet.

Auch bei einer Ausführung des Computertomographen 1 für hohe Energien ist eine dichte Abtastung, d.h. Projektionen aus möglichst Jedem Winkelbereich des Vollkreises, erwünscht. Da in Strahlrichtung hinter den Durchstrahlungstargets 35 jeweils noch ein Ausgleichskörper 36 anzuordnen ist, welcher zwangsläufig auf einem kleineren Radius liegt, kann es bei dichter Anordnung der Durchstrahlungstargets 35 zu Platzproblemen der Ausgleichskörper 36 kommen. Vorliegend sind daher die einzelnen Durchstrahlungstargets 35 um 45° zum Elektronenstrahl 13 geneigt angeordnet.

Die Ausgleichskörper 36, welche in der vorliegenden Darstellung nicht sichtbar sind, sind hierbei in Form eines Dreikants ausgebildet und sind ebenfalls in einem Winkel von 45° in Bezug auf den umlaufenden Elektronenstrahl 13 angeordnet. Durch die Ausbildung in Form eines Dreikants können die Ausgleichskörper 36 trotz einer dichten Anordnung von Durchstrahlungstargets 35 platziert werden. Als Ausgleichskörper wirksam wird hierbei ein Schnitt des Dreikants mit einem Schnittwinkel von 45°. Die Anordnung der Ausgleichskörper 36 in Bezug auf die Durchstrahlungstargets 35 zeigt Fig. 7 in einer schematischen Darstellung, wobei eine Ansicht in Blickrichtung des Pfeils P der Figur 6 dargestellt ist.

Zusätzlich kann das Durchstrahlungstarget 35 quer im Winkel von 45° gescannt werden, wie durch die Linie 35 angedeutet. Zusätzlich zu dem Umlenkvorrichtungen 21 sind bei dieser Ausführung zusätzliche Scanningmagnete (hier nicht dargestellt) zwischen dem umlaufenden Elektronenstrahl 13 und dem Durchstrahlungstarget 35 angeordnet. Die Auflösung des erzeugten Bildes kann hierdurch verbessert werden.

Nach einer weiteren Variante kann auch hierbei der Fokus als Springfokus ausgebildet sein, wobei die Daten In zwei oder auch mehrere Detektorzeilen geschrieben werden. Die Bahnen des Springfokus sind durch die Linien 39 angedeutet.

Somit ist es auch bei Computertomographen 1 mit hohen Energien möglich, den Vollkreis nahezu vollständig abzutasten und die Auflösung zu optimieren.

Die Erfindung ist nicht auf die dargestellten Ausführungsbeispiele beschränkt. Abwandlungen und Kombinationen im Rahmen der Patentansprüche fallen ebenfalls unter die Erfindung.

## Patentansprüche

1. Computertomograph (1) umfassend zumindest eine stationäre Elektronenquelle zur Erzeugung eines Elektronenstrahls (13), eine stationäre Röntgenstrahlenquelle (5) mit einem im wesentlichen ringförmigen Target (14), mit Mitteln zur Führung des Elektronenstrahles (13) in der Röntgenstrahlenquelle (5) auf einer Kreisbahn koaxial zu dem ringförmigen Target (14), sowie mit Mitteln zur Ablenkung (21) des Elektronenstrahls (13) in Richtung auf das Target (14), um ein sich konzentrisch drehendes Röntgenstrahlenbündel (8) zur Durchstrahlung eines Messfeldes (7) aus verschiedenen Richtungen zu erzeugen, und einen ringförmig ausgebildeten, stationären Detektor (6), der aus einer Vielzahl von Detektorelementen besteht, aus deren Messwerten durch einen Rechner ein Bild des Untersuchungsobjektes im Messfeld (7) berechnet wird, wobei das Target (14) eine Vielzahl diskreter Fokusflächen (20) aufweist, welche diskontinuierlich durch einen Fokus des Elektronenstrahls (13') abtastbar sind, **dadurch gekennzeichnet, dass** die Röntgenstrahlenquelle (5) mit dem im wesentlichen ringförmigen Target (14) das Messfeld (7) ringförmig umschließt und das Target (14) eine ungerade Anzahl von Fokusflächen (20) aufweist, welche auf einem Vollkreis verteilt sind, wobei das Target (14) eine gestufte Ringstruktur mit einer ungeraden Anzahl an Sägezahnelementen (23) aufweist oder das Target (14) aus einer ungeraden Vielzahl einzelner Targetelemente besteht, welche jeweils auf einem Sägezahnelement (23) eines gestuften ringförmigen Trägers angeordnet sind.

2. Computertomograph nach dem vorherigen Anspruch, **dadurch gekennzeichnet, dass** die Targetelemente jeweils auswechselbar auf einem Sägezahnelement (23) des gestuften ringförmigen Trägers angeordnet sind.

3. Computertomograph nach Anspruch 1, **dadurch gekennzeichnet, dass** die diskreten Fokusflächen (20) einen Winkel von 58° zur Ebene der Kreisbahn des Elektronenstrahls (13) und/oder einen Anschnittwinkel (α) von 12° zur Achse des ringförmigen Targets (14) aufweisen.

4. Computertomograph nach Anspruch 1, **dadurch gekennzeichnet, dass** der Träger und/oder ein Strahlführungsrohr (12) mehrere separat beaufschlagbare Kühlmittelkanäle (27) aufweisen.

5. Computertomograph nach einem der vorherigen Ansprüche, **dadurch gekennzeichnet, dass** die kinetische Energie der Elektronen des Elektronenstrahls (13) einstellbar ist.

6. Computertomograph nach Anspruch 2, **dadurch gekennzeichnet, dass** die Targetelemente als Durchstrahlungstargets (35) ausgebildet sind.

7. Computertomograph nach dem vorherigen Anspruch, **dadurch gekennzeichnet, dass** in Strahlrichtung nach den Durchstrahlungstargets (35) jeweils ein Ausglelchskörper (36) angeordnet ist.

8. Computertomograph nach einem der vorherigen Ansprüche, **dadurch gekennzeichnet, dass** die Anzahl der Mittel zur Führung der Anzahl der Sägezahnelemente (23) entspricht.

9. Computertomograph nach einem der vorherigen Ansprüche, **dadurch gekennzeichnet, dass** die Mittel zur Ablenkung (21) des Elektronenstrahles (13) durch elektrostatische oder magnetische Kickvorrichtungen an den Mitteln zur Führung des Elektronenstrahls (13) gebildet sind.

10. Computertomograph nach dem vorherigen Anspruch, **dadurch gekennzeichnet, dass** der Ablenkwinkel (β) der Kickvorrichtungen verstellbar ist.

11. Computertomograph nach einem der vorherigen Ansprüche, **dadurch gekennzeichnet, dass** der Fokus des Elektronenstrahls (13') auf den diskreten Fokusflächen (20) verstellbar ist.

12. Computertomograph nach einem der vorherigen Ansprüche, **dadurch gekennzeichnet, dass** der Fokus des Elektronenstrahls (13') als Strichfokus ausgebildet ist, welchem wenigstens eine Zeile des Detektors zugeordnet ist.

13. Computertomograph nach einem der vorherigen Ansprüche, **dadurch gekennzeichnet, dass** der Fokus des Elektronenstrahls (13') als Springfokus ausgebildet ist, welchem wenigstens eine Zeile des Detektors (6) zugeordnet ist, wobei der Fokus an mehrere Plätze der Zeile springt.

14. Computertomograph nach einem der vorherigen Ansprüche, **dadurch gekennzeichnet, dass** die Photonenfluenz einstellbar, vorzugsweise für jede Fokusfläche (20) individuell einstellbar, ist.

15. Computertomograph nach einem der vorherigen Ansprüche, **dadurch gekennzeichnet, dass** die Photonenfluenz durch Regelung der Haltezeit des Elektronenstrahls (13') am einzelnen Fokus, vorzugsweise bei maximaler Elektronenfluenz, einstellbar ist.

16. Computertomograph nach einem der vorherigen Ansprüche, **dadurch gekennzeichnet, dass** der ringförmige Detektor (6) axial versetzt zu dem ringförmigen Target (14) angeordnet ist.

## Claims

1. Computer tomograph (1) comprising at least one stationary electron source for generating an electron beam (13), a stationary X-ray source (5) with a largely ring-shaped target (14) with media for guiding the electron beam (13) in the X-ray source (5) along a circular path coaxially with the ring-shaped target (14), and with media for deflecting (21) the electron beam (13) towards the target (14) in order to generate an X-ray bundle (8) concentrically turning itself to irradiate the measuring field (7) from various directions, and a stationary detector (6) arranged in a ring shape consisting of numerous detector elements from which a computer calculates the measured values in order to create an image of the object being examined in the measuring field (7), the target (14) having numerous discrete focal areas (20) that can be discontinuously scanned through a focal point of the electron beam (13'), **characterized in that** X-ray source (5) with the largely ring-shaped target (14) annularly encloses the measuring field (7) and the target (14) has an odd number of focal areas (20), which are distributed on a full circle, the target (14) having a stepped ring structure with an odd number of saw tooth elements (23) or consisting of an odd large number of individual target elements, which in each case are arranged on a saw tooth element (23) of a stepped ring-shaped carrier.

2. Computer tomograph according to the previous claim **characterized in that** the target elements in each case, are arranged replaceably on a saw tooth element (23) of the stepped ring-shaped carrier.

3. Computer tomograph according to Claim 1 **characterized in that** the discrete focal areas (20) are at an angle of 58° with respect to the plane of the circular path of the electron beam (13) and/or at a lead angle (α) of 12° with respect to the axis of the ring-shaped target (14).

4. Computer tomograph according to Claim 1 **characterized in that** the carrier and/or the beam delivery tube (12) have several cooling channels (27) that can be separately impinged upon.

5. Computer tomograph according to one of the previous claims **characterized in that** the kinetic energy of the electrons of the electron beam (13) can be adjusted

6. Computer tomograph according to Claim 2 **characterized in that** the target elements are executed as irradiation targets (35).

7. Computer tomograph according to the previous claim **characterized in that** in each case a compensating body (36) has been arranged in beam direction after the irradiation targets (35).

8. Computer tomograph according to one of the previous claims **characterized in that** the number of guiding media corresponds to the number of saw tooth elements (23).

9. Computer tomograph according to one of the previous claims **characterized in that** the deflecting media (21) of the electron beam (13) are formed by electrostatically or magnetic kicker devices located in the guiding media of the electron beam (13).

10. Computer tomograph according to the previous claim **characterized in that** the deflecting angle (β) of the kicker devices is adjustable.

11. Computer tomograph according to one of the previous claims **characterized in that** the focal point of the electron beam (13') on the discrete focal areas (20) is adjustable.

12. Computer tomograph according to one of the previous claims **characterized in that** the focal point of the electron beam (13') has been executed as line focus to which at least one line of the detector has been allocated.

13. Computer tomograph according to one of the previous claims **characterized in that** the focal point of the electron beam (13') has been executed as a spring focus to which at least one line of the detector (6) has been allocated, in which case the focal point jumps to several spots of the line.

14. Computer tomograph according to one of the previous claims **characterized in that** the photon fluence is adjustable, preferably individually adjustable for every focal area (20).

15. Computer tomograph according to one of the previous claims **characterized in that** the photon fluence is adjustable by regulating the stopping time of the electron beam (13') in the individual focal point, preferably at maximum electron fluence.

16. Computer tomograph according to one of the previous claims **characterized in that** the ring-shaped detector (6) has been arranged in an axially displaced way with respect to the ring-shaped target (14).

## Revendications

1. Tomographe assisté par ordinateur (1) comprenant au moins une source d'électrons stationnaire pour générer un faisceau électronique (13), une source de rayons X stationnaire (5) avec une cible essentiellement annulaire (14), avec des moyens pour le guidage du faisceau électronique (13) dans la source de rayons X (5) sur une trajectoire circulaire coaxiale à la cible annulaire (14), ainsi que des moyens pour la déviation (21) du faisceau électronique (13) dans la direction de la cible (14) afin de générer un faisceau de rayons X (8) en rotation concentrique pour irradier un champ de mesure (7) à partir de directions différentes, et un détecteur (6) stationnaire se présentant sous forme annulaire, qui se compose d'une multitude d'éléments détecteurs, dont les valeurs de mesure servent à un calculateur pour calculer une image de l'objet de l'examen dans le champ de mesure (7), sachant que la cible (14) présente une multitude de surface focales discrètes (20), qui peuvent être balayées en discontinu par un focus du faisceau électronique (13'), **caractérisé en ce que** la source de rayons X (5) avec la cible essentiellement annulaire (14) enferme le champ de mesure (7) de manière annulaire et la cible (14) présente un nombre impair de surfaces focales (20), qui sont réparties sur un cercle entier, sachant que la cible (14) présente une structure annulaire étagée avec un nombre impair d'éléments en dents de scie (23) ou la cible (14) se compose d'un nombre impair d'éléments-cibles individuels, qui sont disposés respectivement sur un élément en dent de scie (23) d'un support annulaire étagé.

2. Tomographe assisté par ordinateur selon la revendication précédente, **caractérisé en ce que** les éléments-cibles sont respectivement disposés de manière remplaçable sur un élément en dent de scie (23) du support annulaire étagé.

3. Tomographe assisté par ordinateur selon la revendication 1, **caractérisé en ce que** les surfaces focales discrètes (20) présentent un angle de 58° par rapport au plan de la trajectoire circulaire du faisceau électronique (13) et/ou un angle de coupure (α) de 12° par rapport à l'axe de la cible annulaire (14).

4. Tomographe assisté par ordinateur selon la revendication 1, **caractérisé en ce que** le support et/ou un tube de guidage de faisceau (12) présente(nt) plusieurs canaux d'agent réfrigérant (27) pouvant être alimentés séparément.

5. Tomographe assisté par ordinateur selon l'une quelconque des revendications précédentes, **caractérisé en ce que** l'énergie cinétique des électrons du faisceau électronique (13) est réglable.

6. Tomographe assisté par ordinateur selon la revendication 2, **caractérisé en ce que** les éléments-cibles se présentent sous la forme de cibles à radiographie (35).

7. Tomographe assisté par ordinateur selon la revendication précédente, **caractérisé en ce qu'**un corps de compensation (36) est disposé respectivement en aval des cibles à radiographie (35) dans le sens du rayonnement.

8. Tomographe assisté par ordinateur selon l'une quelconque des revendications précédentes, **caractérisé en ce que** le nombre des moyens pour le guidage correspond au nombre d'éléments en dents de scie (23).

9. Tomographe assisté par ordinateur selon l'une quelconque des revendications précédentes, **caractérisé en ce que** les moyens pour la déviation (21) du faisceau électronique (13) sont formés aux moyens pour le guidage du faisceau électronique (13) par des dispositifs de déflexion rapide électrostatiques ou magnétiques.

10. Tomographe assisté par ordinateur selon la revendication précédente, **caractérisé en ce que** l'angle de déflexion (β) des dispositifs de déflexion rapide est réglable.

11. Tomographe assisté par ordinateur selon l'une quelconque des revendications précédentes, **caractérisé en ce que** le foyer électronique du faisceau électronique (13') sur les surfaces focales (20) est réglable.

12. Tomographe assisté par ordinateur selon l'une quelconque des revendications précédentes, **caractérisé en ce que** le foyer électronique du faisceau électronique (13') se présente sous la forme d'un foyer linéaire, auquel est attribué au moins une ligne du détecteur.

13. Tomographe assisté par ordinateur selon l'une quelconque des revendications précédentes, **caractérisé en ce que** le foyer électronique du faisceau électronique (13') se présente sous la forme d'un foyer flottant, auquel est attribué au moins une ligne du détecteur (6), sachant que le foyer électronique saute à plusieurs emplacements de la ligne.

14. Tomographe assisté par ordinateur selon l'une quelconque des revendications précédentes, **caractérisé en ce que** la fluence photonique est réglable, de préférence réglable individuellement pour chaque surface focale (20)

15. Tomographe assisté par ordinateur selon l'une quelconque des revendications précédentes, **caractérisé en ce que** la fluence photonique est réglable par réglage du temps de maintien du faisceau électronique (13') à un foyer individuel, de préférence à la fluence électronique maximale.

16. Tomographe assisté par ordinateur selon l'une quelconque des revendications précédentes, **caractérisé en ce que** le détecteur annulaire (6) est disposé à une position axialement décalée par rapport à celle de la cible annulaire (14).
